# EUROPEAN PATENT APPLICATION

(11) **EP 1 229 041 A1**
(43) Date of publication of application: **07.08.2002**
(21) Application number: 01102490.8
(22) Date of filing: 05.02.2001
(51) Int. Cl.: C07J 41/00

(54) **A process for the preparation of 3-Glutamido bile ester derivatives using N-tBoc methyl pyroglutamate**

(71) Applicant: Bracco Imaging S.p.A., 20134 Milano (IT)
(72) Inventor: Brochetta, Marino, 20134 Milano (IT); Visigalli, Massimo, 20134 Milano (IT); Palano, Daniela, 20134 Milano (IT); Manfredi, Giuseppe, 20134 Milano (IT); Alessandroni, Laura, 20134 Milano (IT); Anelli, Pier Lucio, 20134 Milano (IT)
(74) Representative: Minoja, Fabrizio, Dr.

(57) **Abstract**

The present invention relates to a novel process for the preparation of bile esters derivatives of general formula (I), in which R₁ is H or OH; R₂ is H, α-OH or β-OH and R₃ is a straight or branched C₁-C₄ alkyl group or a benzyl group.

The process uses either L-glutamic acid hydrochloride (II) or L-glutamic acid as starting material, according to the following Scheme 1.

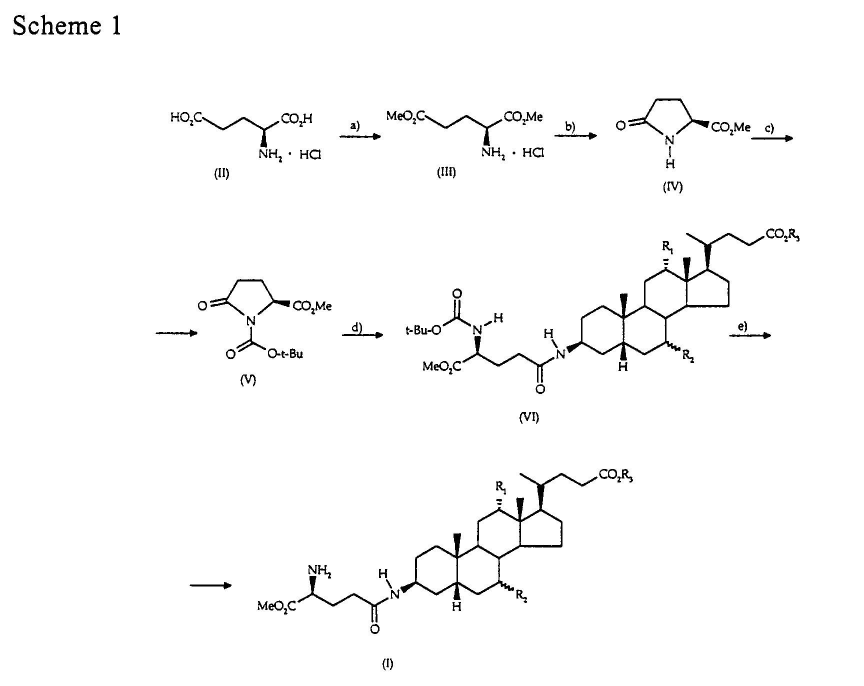

## Description

The present invention relates to a novel process for the preparation of intermediates used for the preparation of contrast agents.

More particularly, the present invention relates to a process for the preparation of bile esters derivatives of general formula (I) 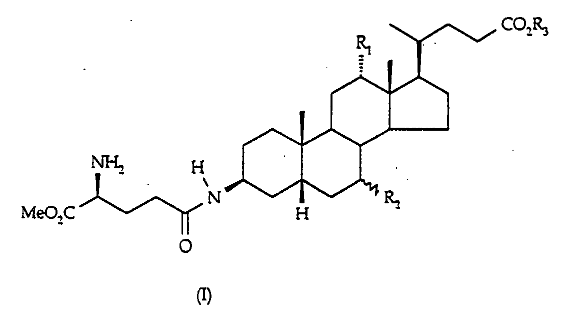 wherein:
R₁ is H or OH,
R₂ is H, α-OH or β-OH, and
R₃ is a straight or branched C₁-C₄ alkyl group or a benzyl group,
   starting from either L-glutamic acid hydrochloride (II) or L-glutamic acid.

The compounds of formula (I) are intermediates used in the preparation of contrast agents, whose use in nuclear magnetic resonance diagnostics was extensively described in WO/0038738. The latter reports the synthesis of the compounds of formula (I), through the intermediates of formula (VII), according to the following Scheme 2: 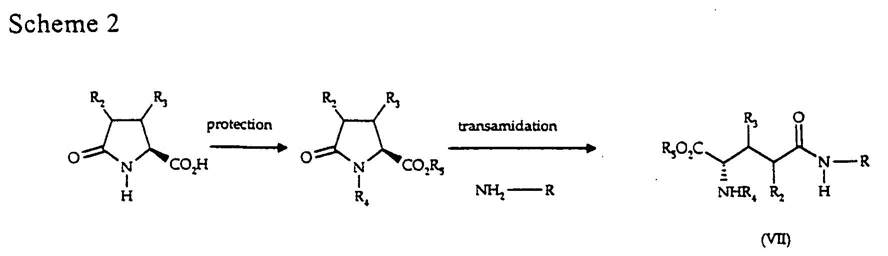 wherein:
- R₄: is an amino-protecting group;
- R₅: is a straight or branched C₁-C₁₀ alkyl or aryl,
- R₂ and R₃: are independently an hydrogen atom, straight or branched C₁-C₂₀ alkyl, unsubstituted or substituted by aryl groups, or said groups form a C₃-C₁₀ ring
- and R: is the reactive derivative of the convenient bile acid.

The transamidation reaction maintains the stereochemistry at the chiral centre adjacent to the nitrogen atom of the starting pyrrolidinone and affords a secondary amide. The combined selection of R₄ and R₅ groups is important in that the cleavage should take place under diversified conditions. In the same patent application the use of (*S*)-5-oxo-1,2-pyrrolidinedicarboxylic acid 2-methyl 1-(phenylmethyl) diester (VIII), *i.e.* the compound bearing a carbobenzyloxy (Cbz) group as R₄, has been exemplified.

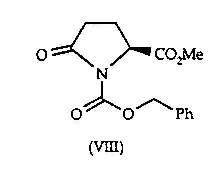

The previous process involves some drawbacks which should be possibly overcome before an industrial scale-up:
- the deprotection step involves the use of hydrogen and a catalyst;
- the intermediate (VIII) is an oil which is not stored and, accordingly, is prepared just before use.

We have surprisingly found that, with a few modifications to the process of Scheme 2, the above discussed technical problems can be successfully solved.

The present invention therefore relates to a process for the preparation of compounds of general formula (I), 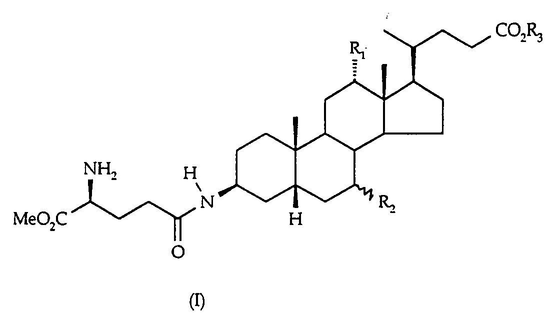 wherein:
- R₁: is H or OH,
- R₂: is H, α-OH or β-OH, and
- R₃: is a straight or branched C₁-C₄ alkyl group or a benzyl group,
which process consists in:
a) esterifying either L-glutamic acid hydrochloride (II) or L-glutamic acid to obtain L-glutamic acid dimethyl ester hydrochloride (III): 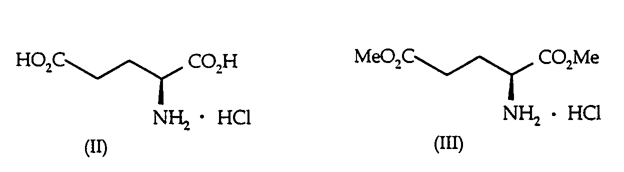
b) subjecting the resulting ester (III) to intramolecular amidation by treatment with bases, followed by heating, to give L-5-oxoproline methyl ester (IV): 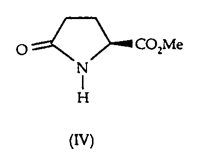
c) protecting the lactam nitrogen of (IV) by means of a t-butoxycarbonyl group to give the compound of formula (V), *(S)*-5-oxo-1,2-pyrrolidinedicarboxylic acid 1-(1,1-dimethylethyl) 2-methyl ester: 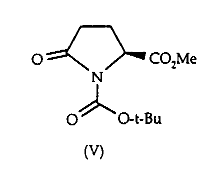
d) subjecting the compound of formula (V) to transamidation, by treatment with a compound of general formula (IX): 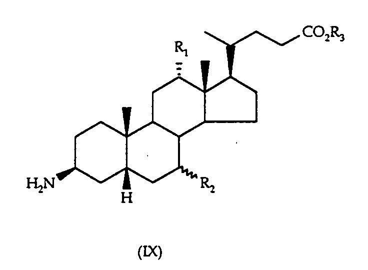 wherein R₁, R₂ and R₃ are as defined above,
   to give a compound of formula (VI): 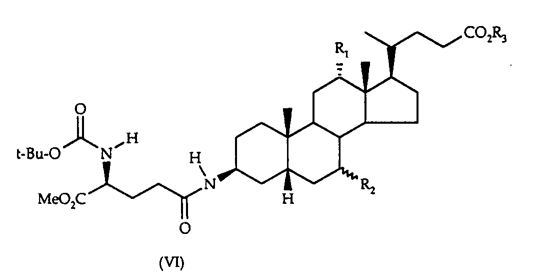
e) deprotection of compounds of formula (VI), to give the compounds of general formula (I).

The process of the invention is summarised in the following Scheme 1:

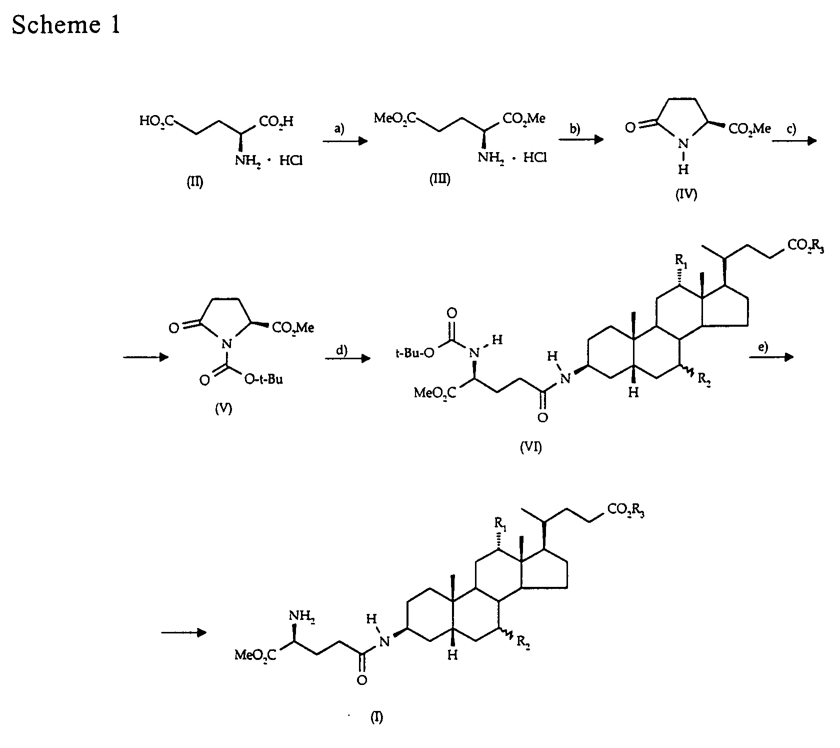

The compounds of formula (IX), are prepared according to the methods disclosed in WO-A-95/32741 or in PCT/EP00/08226. These compounds are bile acid derivatives in which a 13-amino group is always present in position 3.

The most important examples of bile acids of the present invention are selected from the group consisting of residues of cholic, chenodeoxycholic, deoxycholic, ursodeoxycholic, lithocholic acids represented by the following formulae.

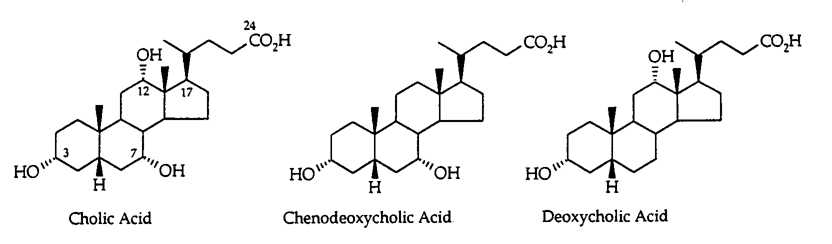

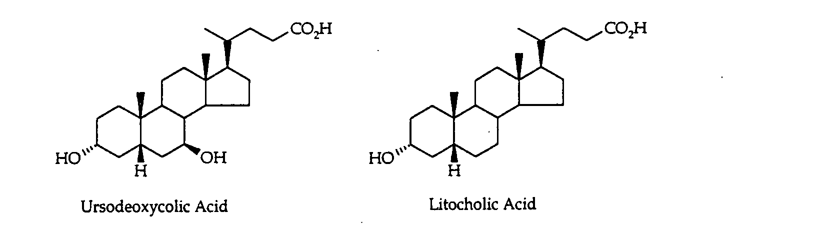

The process of Scheme 1 is characterized by the use of the intermediate of formula (V), *(S)*-5-oxo-1,2-pyrrolidinedicarboxylic acid 1-(1,1-dimethylethyl) 2-methyl ester.

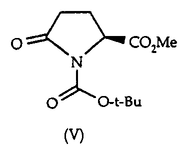

The latter is a known intermediate used for the preparation of pharmaceuticals, *e.g.* antitumoral agents (JP05247047; Tetrahedron, 1994, 50, 6221-6238).

The preparation of compound (V) (see for example J. Org. Chem., 1999, 64, 6646-6652; Synlett, 1995, 519-522; Synth. Comm, 1995, 25, 4045-4052; Tetrahedron, 1994, 50, 6221-6238; Tetrahedron Lett., 1993, 34, 5743-5746) preferably uses *(S)*-5-oxoproline (X) as starting material, with a synthetic pathway described in the following Scheme 3.

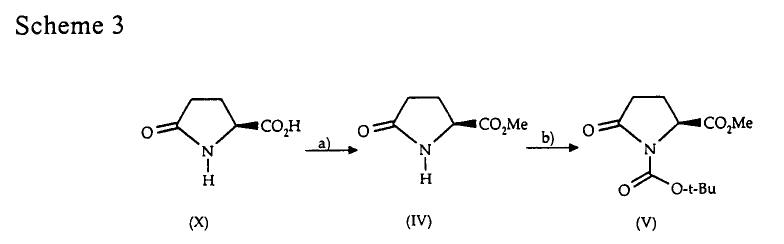

The subsequent protection of *(S)*-5-oxoproline methyl ester (IV) is reported for example in: JP05247047; Eur. J. Org. Chem., 1999, 1581-1584; Tetrahedron Lett., 1998, 39, 4789-4792; Tetrahedron Lett., 1993, 34, 5455-5458; Chem. Pharm. Bull., 1991, 39, 1199-1212; J. Org. Chem. 1983, 2424-2426.

As described in Scheme 1, in step a) either L-glutamic acid hydrochloride (II) or L-glutamic acid are esterified to give L-glutamic acid dimethyl ester hydrochloride (III). This reaction may be performed by known esterification methods for carboxylic acids.

In particular, compound (II) is first suspended in methanol and then dissolved by adding at least 2 moles of SOCl₂ per mole of (II).The temperature is maintained at 0-5°C during the addition, and then the reaction is completed in 23 hours at 23°C. The thick oil obtained after the evaporation of the solvent is directly used, without any purification, in the next step.

In step b) compound of formula (III) is first neutralized with 3 M KOH in methanol solution, the precipitated KCl is filtered off. The filtrate is evaporated and then heated for 1 to 7 hours, to obtain *(S)*-5-oxoproline methyl ester (IV). The heating time depends on the temperature used during the cyclization reaction, which can vary from 80 to 130°C. Compound (IV) can be used directly in the subsequent step c), or it can be purified, as described in Chem. Pharm. Bull., 1990, 28(5), 1449-1458, by distillation at reduced pressure (0.2 kPa). However, these conditions are not easily achieved at industrial level and, moreover, the distillation of large quantities would require a long time. The prolonged heating at high temperature would bring about a decrease in enantiomeric excess and a yield loss. As a matter of fact, even at lab scale, the final distillation fractions showed a content of the *R* enantiomer ranging from 2.5 up to 7.9 %.

In step c) the lactam nitrogen of (IV) is protected with the *t*-butoxycarbonyl group according to known methods. This reaction is preferably carried out by adding at least one mole of di-t-butyl dicarbonate, to a solution containing one mole of (IV) in a solvent selected in the group consisting of: C₁-C₅ alkyl esters of acetic acid, aprotic dipolar solvents, CH₃CN, aromatic solvents like toluene. EtOAc and CH₃CN are preferred. The reaction is usually catalyzed by addition of, for example, 4-(dimethylamino)pyridine (DMAP), in a quantity ranging from 0.01 to 0.1 moles per mole of (IV).

The reaction is completed in two hours at room temperature and, after evaporation of the solvent, the crude residue of (IV) is dissolved in EtOAc. The solution is washed with aq. pH 5.7 phosphate buffer, and water, then dried (Na₂SO₄) and purified by crystallization from a solvent, preferably EtOAc and/or *n*-hexane.

The product isolated in 88% yield, is a solid that can be stored for months without degradation.

In step d) one mole of compound of formula (IX) is reacted with 1 to 1.5 mole of compound (V), in a solvent, selected from *N,N*-dimethylacetamide, *N,N*-dimethylformamide, EtOAc, BuOAc, toluene, xylene, p-cymene, diethylbenzene or similar aromatic solvent substituted with one or more linear or branched alkyl groups. The reaction mixture is stirred for 12 to 30 hours at a temperature from 70 to 130°C depending on the reagents and solvent used.

Once the reaction is completed and after spontaneous cooling to room temperature, the precipitate is filtered off, washed and dried, to afford the condensation product of formula (VI).

In the subsequent step e), compound of formula (VI) is subjected to acidic hydrolysis to remove the *t*-butoxycarbonyl group, to give the final compound of general formula (I).

The reaction preferably consists in a slow addition of an inorganic or organic acid, under anhydrous (e.g. gas) or aqueous form, to a solution of the compound (VI) in a suitable alcohol or C₁-C₄ alkyl ester of acetic acid, maintaining a reaction temperature from 15 to 60°C.

The resulting solution is kept at a temperature from 15 to 60°C for a time from 0.5 to 20 hours, wherein temperature and time of reaction depend on the compound used in the conversion of (VI) to (I).

In this step the acid compound is preferably selected from HCl gas, aq. HCl, aq. H₂SO₄, CF₃CO₂H, CH₃CO₂H, oxalic acid, methanesulfonic acid or *p*-toluenesulfonic acid. The acid is added in a quantity corresponding to 1-3 moles per mole of (VI).

The compound of formula (I) is isolated either as a salt of the previously used acid or as a free amine of general formula (I).

The isolation of (I) as a free amine, consists in a preliminary neutralization of the acidic mixture obtained at the end of reaction, by adding a base preferably selected from tertiary amines such as triethylamine (TEA) or diisopropylethylamine (DIEA). Compound (I) is isolated in this step in a yield ranging from 80 to 97 %.

The use of a hindered tertiary amine affords the isolation of compounds (I) minimizing the formation of by products due to secondary reactions, such as hydrolysis of the ester groups or transamidation. In fact aqueous bases, such as aq. NaOH or KOH, can hydrolyse the ester groups, and in particular the ester group present on the glutamic chain which is easier to cleave than the ester group in the cholanoic moiety. Furthermore, the use of a NH₃ solution or of primary or secondary amines can promote the transamidation reaction.

The process for the preparation of (3β,5β,12α)-3-[[4(*S*)-4-amino-5-methoxy-1,5-dioxo]pentyl]amino]-12-hydroxycholan-24-oic acid methyl ester, compound of formula (I A), according to Scheme 4, is particularly preferred.

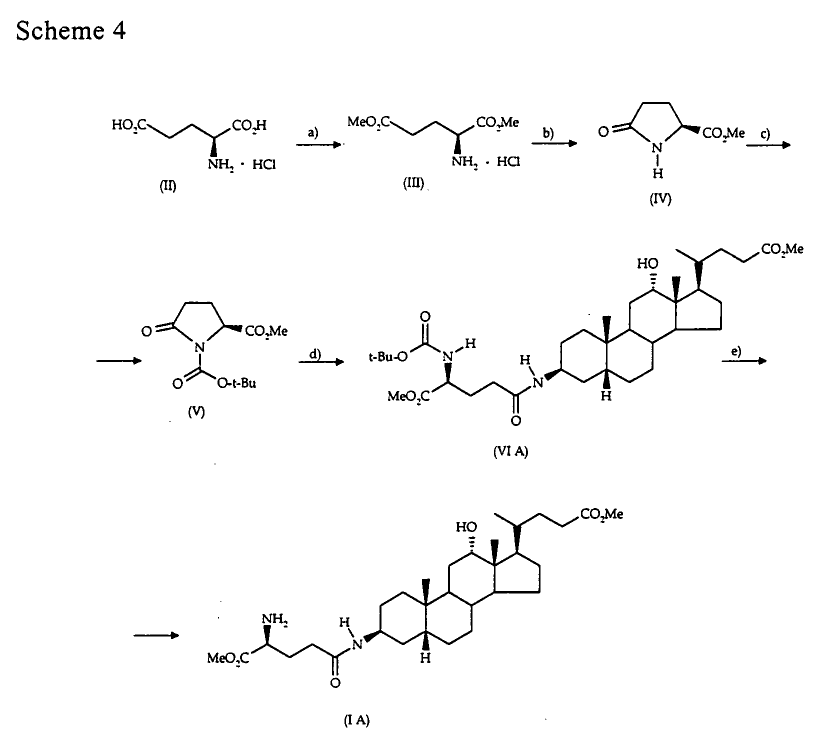

In step a), compound (II) is first suspended in methanol and then dissolved by adding at least 2 moles of SOCl₂ per mole of (II). The temperature is maintained at 0-5°C during the addition, and then the reaction is completed in 20 hours at 23°C.

The thick oil obtained after the evaporation of the solvent is directly used, without any purification, in the next step.

In step b), compound of formula (III) is first neutralized with 3 M KOH in methanol solution, then the precipitated KCl is filtered off. The filtrate is evaporated and then heated for 1 hour, at a temperature varying from 110 to 115°C. The resulting *(S)*-5-oxoproline methyl ester (IV) is directly used in the next step c).

In step c), at least one mole of di-*t*-butyl dicarbonate is added to a solution containing one mole of (IV), in a solvent selected in the group consisting of: C₁-C₅ alkyl esters of acetic acid, aprotic dipolar solvents, CH₃CN, aromatic solvents like toluene. EtOAc and CH₃CN are the preferred ones. The reaction is usually catalyzed by addition of, for example, 4-(dimethylamino)pyridine (DMAP), in a quantity ranging from 0.01 to 0.1 moles per mole of (IV).

The reaction is completed in two hours at room temperature and, after evaporation of the solvent, the crude residue of (IV) is dissolved in EtOAc. The solution is washed with aq. pH 5.7 phosphate buffer, and water, then dried (Na₂SO₄) and purified by crystallization from a solvent, preferably EtOAc and/or *n*-hexane.

The product isolated in 88% yield, is a solid that can be stored for months without degradation.

In step d) (3β,5β,12α)-12-hydroxy-3-[[5-methoxy-1,5-dioxo-4(*S*)-4-[[(1,1-dimethylethoxy)carbonyl]amino]pentyl]amino]cholan-24-oic acid methyl ester of formula (VI A) is obtained by reaction of *(S)*-5-oxo-1,2-pyrrolidine-dicarboxylic acid 1-(1,1-dimethylethyl) 2-methyl ester (V), with (3β,5β,12α)-3-amino-12-hydroxycholan-24-oic acid methyl ester (XI), prepared according to the method described in WO-A-95/32741 or in the patent application PCT/EP00/08226 of the same assignee.

The preferred process conditions to afford compound (VI A) in step d), consists in mixing a suspension of one mole of (XI) with at least one mole of compound (V), in a solvent preferably selected from: toluene, xylene, C₁-C₄ alkyl esters of acetic acid, *N,N*-dimetylacetamide, p-cymene or diethylbenzene.

The reaction mixture is kept at a temperature from 80 to 110°C and for a time of 20 to 30 hours, necessary for a complete conversion of the reagents.

Once the reaction is completed the solution is cooled to room temperature during 15 hours and then the precipitate, is filtered off and washed. The product is isolated in a yield ranging from 72 to 78%.

In Step e) compound (VI A) is hydrolysed to compound of formula (I A) by slow addition of 1 to 3 moles of HCl or methanesulfonic acid to a methanol solution of compound (VI A), below 20°C. The complete conversion of (VI A) to (I A) is obtained by keeping the solution for 24 hours at room temperature.

Compound (I A) can be isolated as a salt of the previously used acid, or as an amine in a yield from 88 to 92 %.

The isolation of (I A) as free amine is obtained by neutralizing the solution with an addition of a tertiary amine, preferably selected from triethylamine (TEA) or diisopropylethylamine (DIEA). Otherwise the isolation of (I) as a salt is obtained through crystallization or insolubilization.

The experimental conditions used will be illustrated in detail in the following experimental section.

### EXPERIMENTAL SECTION

### Thin Layer Chromatography:

Silica gel plates used for the TLC are: 60 F₂₅₄
Eluent A: 70 : 25 : 3 CHCl₃/MeOH/25% NH₄OH
Eluent B: 80 : 20 EtOAc/CH₂Cl₂
Detection : exposure to Cl₂ vapours + o-tolidine

### Analytical HPLC methods:

| *Method A* | |
|---|---|
| Stationary phase | Chiralcel OD-H, 250 x 4.6 mm column packed by Daicel |
| Temperature | 40°C |
| Mobile phase | isocratic elution: A/B = 93 : 7 A = *n*-hexane, B = ethanol |
| Flow rate | 1.0 mL min⁻¹ |
| Detection (UV) | 210 nm |
| Injection | 20 µL |
| Sample concentration | 2.0 mg mL⁻¹ (racemic mixture), 5.0 mg mL⁻¹ (optically active) |

| *Method B* | | | |
|---|---|---|---|
| Stationary phase | Lichrosorb RP-Select B 5 µm, 250 x 4 mm column packed by Merck KGaA | | |
| Temperature | 45°C | | |
| Mobile phase | gradient elution, A = 0.017 M H₃PO₄ in water, B = CH₃CN | | |
| Gradient timetable | min | % A | % B |
| | 0 | 82 | 18 |
| | 30 | 15 | 85 |
| | 45 | 15 | 85 |
| Flow rate | 1 mL min⁻¹ | | |
| Detection (UV) | 210 nm | | |
| Injection | 10 µL | | |
| Sample concentration | 2 mg mL⁻¹ | | |

| *Method C* | |
|---|---|
| Stationary phase | Chiralcel OD-H, 250 x 4.6 mm column packed by Daicel |
| Temperature | 40°C |
| Mobile phase | isocratic elution: A/B = 95 : 5 A = *n*-hexane, B = ethanol |
| Flow rate | 1.0 mL min⁻¹ |
| Detection (UV) | 210 nm |
| Injection | 20 µL |
| Sample concentration | 0.4 mg mL⁻¹ (racemic mixture), 1.0 mg mL⁻¹ (optically active) |

| *Method D* | |
|---|---|
| Stationary phase | Chiralcel OD-H; 250 x 4.6 mm column packed by Daicel; |
| Temperature | 40°C; |
| Mobile phase | isocratic elution: A/B = 92:8; A = *n*-hexane, B = ethanol |
| Flow rate | 1.0 mL min⁻¹; |
| Detection (UV) | 210 nm; |
| Injection | 10 µL; |
| Sample concentration | 2.0 mg mL⁻¹ (racemic mixture), 5.0 mg mL⁻¹ (optically active); |

| *Method E* | | | |
|---|---|---|---|
| Stationary phase | Lichrosorb RP-Select B 5 µm; 250 x 4 mm column packed by Merck KGaA; | | |
| Temperature | 45°C; | | |
| Mobile phase | gradient elution; A = 0.017 M H₃PO₄ in water, B = CH₃CN | | |
| Gradient timetable | min | % A | % B |
| | 0 | 82 | 18 |
| | 30 | 15 | 85 |
| | 45 | 15 | 85 |
| Flow rate | 1 mL min⁻¹; | | |
| Detection (UV) | 210 nm; | | |
| Injection | 10 µL; | | |
| Sample concentration | 1 mg mL⁻¹ | | |

| *Method F* | |
|---|---|
| Stationary phase | Chiralcel OD; 250 x 4.6 mm column packed by Daicel; |
| Temperature | 40°C; |
| Mobile phase | isocratic elution: A/B = 85:15; A = *n*-hexane, B = 2-propanol |
| Flow rate | 1.0 mL min⁻¹; |
| Detection (UV) | 210 nm; |
| Injection | 20 µL; |
| Sample concentration | 0.7 mg mL⁻¹ (racemic mixture), 3.0 mg mL⁻¹ (optically active); |

### Example 1

### Preparation of L-glutamic acid dimethyl ester hydrochloride (III)

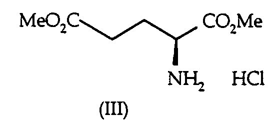

SOCl₂ (732 g; 6.15 mol) was added over 2 h to a suspension of either L-glutamic acid hydrochloride (II) (551 g; 3 mol) or L-glutamic acid (441.4 g; 3 mol) in MeOH (3.5 L) stirred at 0-5°C. After about 3.5 h at r.t. the reaction mixture turned into a clear solution that was stirred for 20 h. The solvent was evaporated to give (III) (650.8 g) as a thick oil that was used in the following step without any purification.
TLC : Rf 0.79 (Eluent A)
Argentometric titer (0.1 N AgNO₃): 105.3 %
The ¹H-NMR, ¹³C-NMR, and MS spectra are consistent with the indicated structure.

### Example 2

### Preparation of L-5-oxoproline methyl ester (IV)

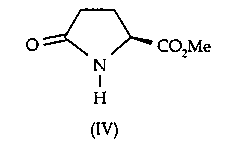

3 M KOH in MeOH (1.08 L; 3.24 mol) was added over 0.5 h to a solution of (III) (650.6 g), prepared as described in example 1, in MeOH (1 L) to precipitate KCl that was filtered off. The clear solution was concentrated, filtered (as the precipitation of further KCl occurred) and evaporated. The residue was heated at 115°C at atmospheric pressure for about 1 h while distilling MeOH produced by the cyclization reaction to obtain crude (IV) (447 g) as a colourless oil which was used in the next step without purification.
TLC : Rf 0.71 (Eluent A)
HPLC : S/R ratio 99.0 : 1.0 (Method A)
The ¹H-NMR, ¹³C-NMR, and MS spectra are consistent with the indicated structure.

### Example 3

### Preparation of (S)-5-oxo-1,2-pyrrolidinedicarboxylic acid 1-(1,1-dimethylethyl) 2-methyl ester (V)

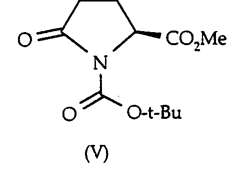

Di-t-butyl dicarbonate (611 g; 2.8 mol) was added over 1 h to a cloudy solution of (IV) (447 g), preparared as described in example 2, and 4-(dimethylamino)pyridine (DMAP) (6.1 g; 0.05 mol) in CH₃CN (2.8 L) stirred at 15-18°C. After 2 h the solvent was evaporated. The residue was dissolved in EtOAc, washed with aq. pH 5.7 phosphate buffer and H₂O. After drying, the solvent was evaporated to give crude (V) as a thick oil. The latter was dissolved in EtOAc and the solution slowly diluted with *n*-hexane to induce crystallisation. After 15 h at r.t. the solid was filtered, washed with *n*-hexane and dried to afford (V) (474.3 g; 1.95 mol) as a white solid.

Yield 65 %. The mother liquors and the washings were combined and evaporated. The oily residue was treated with *n*-hexane to give a second crop of (V) (73 g; 0.3 mol) (yield 10 %) as a whitish solid of purity similar to the one of the first crop. Overall yield (from II) 75 %.
mp : 70-71.5°C
TLC : Rf 0.74 (Eluent B)

- HPLC (method B):: first crop 99.6 % (area %)
second crop 98.8 % (area %)
- HPLC (method C): S/R ratio: first crop 100 : 0
second crop 100 : 0
The ¹H-NMR, ¹³C-NMR, IR and MS spectra are consistent with the indicated structure.

### Example 4

### Preparation of (3β,5β,12α)-12-hydroxy-3-[[5-methoxy-1,5-dioxo-4(S)-4-[[(1,1-dimethylethoxy)carbonyl]amino]pentyl]amino]cholan-24-oic acid methyl ester (VI A)

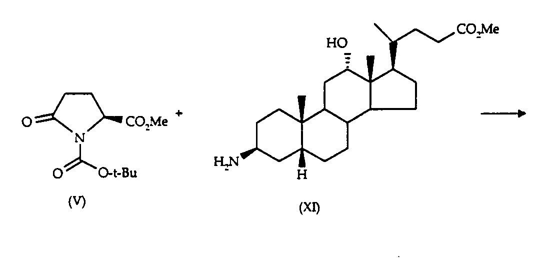

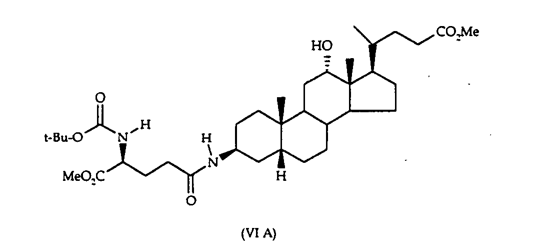

A suspension of (XI) (625 g; 1.54 mol) and (V) (374.6 g; 1.54 mol) in toluene (1.54 L) was stirred at 90°C for 24 h. After spontaneous cooling to r.t. over 15 h, the precipitate was filtered off, washed with toluene and dried (40°C; 2 kPa) to afford (VI A) as a 1:1 clatrate with toluene (889.2 g; 1.2 mol).
Yield 78 %.
HPLC (method D): e.e.> 99.6 %
HPLC (method E): 98 %
The ¹H-NMR, ¹³C-NMR, IR and MS spectra are consistent with the indicated structure.

### Example 5

### Preparation of (3β,5β,12α)-3-[[4(S)-4-amino-5-methoxy-1,5-dioxopentyl]amino]-12-hydroxycholan-24-oic acid methyl ester (I A)

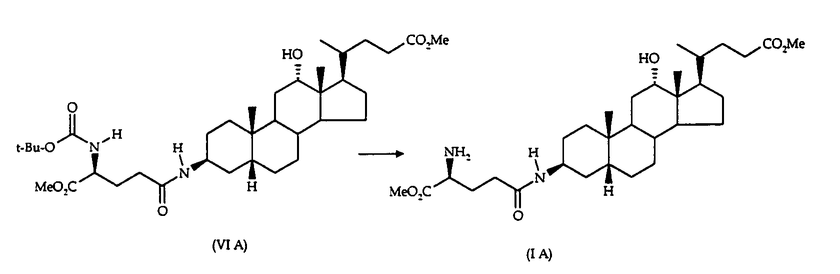

To a solution of compound (VI A) 1:1 clatrate with toluene (231.6 g; 0.31 mol) prepared as described in example 4, in MeOH (1.16 L), methanesulfonic acid (56.7 g; 0.6 mol) was slowly added while maintaining the reaction temperature below 20°C. The resulting solution was stirred at r.t. for 24 h. Then DIEA (77.6 g; 0.6 mol) was added and the solution was evaporated to a crude residue that was taken up with water. After one hour stirring at r.t. the solid was filtered, washed with water and dried to afford (I A) (149.1 g; 0.27 mol) as a white solid. Yield 87 %.
HPLC (method E): 98.4 % (area %)
HPLC (method F): e.e.> 99.5%
The ¹H-NMR, ¹³C-NMR, IR and MS spectra are consistent with the indicated structure.

### Example 6

### Preparation and isolation of (I A) as dihydrocloride

A solution of compound (VI A) 1:1 clatrate with toluene (30 g; 40 mmol) prepared as described in example 4, in 2.5 M HCl in MeOH (100 mL) was stirred at r.t. for 15 h then the solution was seeded. After 2 h at 0°C, the solid was filtered, washed with cold 1.5 M HCl in MeOH (30 mL) and dried to obtain (I A) hydrochloride (21.4 g; 34.4 mmol) containing a further mole of HCl as a white solid.
Yield 86 %.
TLC: (eluent B) Rf 0.68
HPLC (method E) : 96.9 % (area %)
HPLC (method F) : e.e.> 99.5 %
Argentometric titer (0.1 N AgNO₃) : 97.3 %
The ¹H-NMR, ¹³C-NMR, IR and MS spectra are consistent with the indicated structure.

## Claims

1. A process for the preparation of bile ester derivatives of general formula (I), 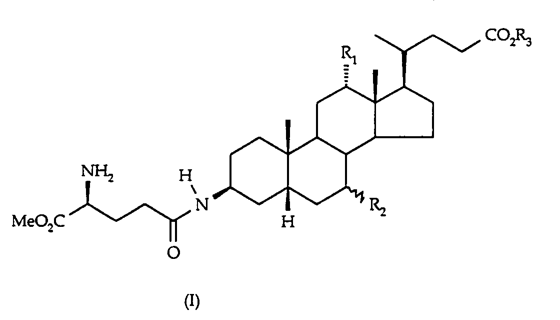 wherein:
R₁ is H or OH,
R₂ is H, α-OH or β-OH, and
R₃ is a straight or branched C₁-C₄ alkyl group or a benzyl group,
which process consists in:
a) esterifying either L-glutamic acid hydrochloride (II) or L-glutamic acid to obtain L-glutamic acid dimethyl ester hydrochloride (III): 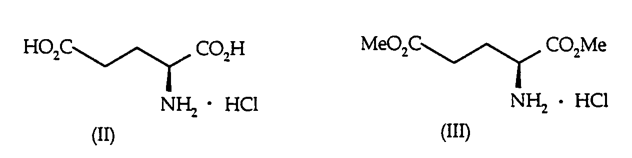
b) subjecting the resulting ester (III) to intramolecular amidation by treatment with bases, followed by heating, to give the cyclic compound L-5-oxoproline methyl ester (IV): 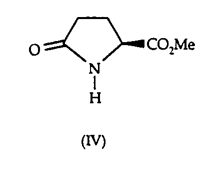
c) protecting the lactam nitrogen of (IV) by means of a t-butoxycarbonyl group to give the compound of formula (V), *(S)*-5-oxo-1,2-pyrrolidinedicarboxylic acid 1-(1,1-dimethylethyl) 2-methyl ester: 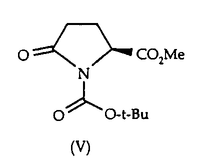
d) subjecting the compound of formula (V) to transamidation, by treatment with a compound of general formula (IX): 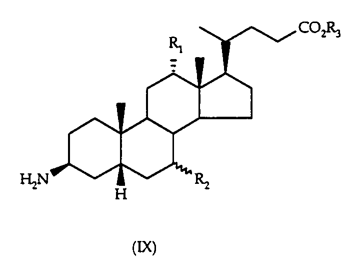 wherein R₁, R₂ and R₃ are as defined above,
to give a compound of formula (VI): 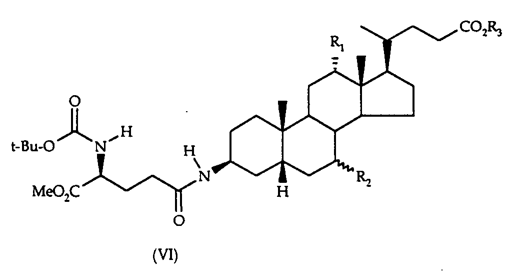
e) hydrolysing compounds of formula (VI) under acidic conditions, to give the compounds of general formula (I).

2. A process according to claim 1, in which the compounds of general formula (IX) are derivatives of the following bile acids:

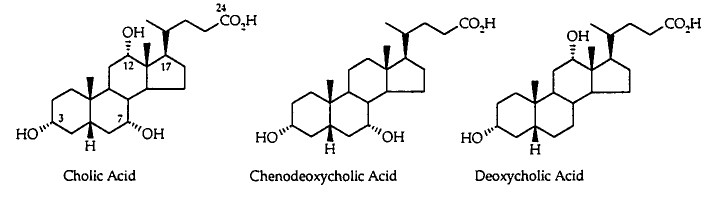

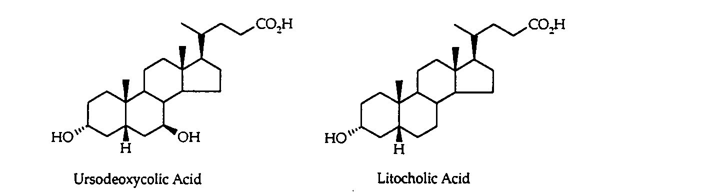

3. A process according to claims 1-2, in which:
• step a) is carried out in methanol in the presence of at least 2 mole of SOCl₂ per mole of (II);
• step b) is carried out using a base;
• step c) is carried out using at least one mole of di-t-butyl dicarbonate per mole of compound (IV) in the presence of 0.01 to 0.1 mol of 4-(dimethylamino)-pyridine per mole of compound (IV), in a solvent selected from: C₁-C₅ alkyl ester of acetic acid, aprotic dipolar solvents, CH₃CN and aromatic solvents;
• step d) is carried out using one mole of compound (IX) per 1 to 1.5 mole of compound (V), in a solvent selected from: *N,N*-dimethyl-acetamide, N,N-dimethylformamide, EtOAc, BuOAc, toluene, xylene, p-cymene, diethylbenzene or another aromatic solvent substituted with one or more alkyl linear or branched group; at a temperature from 70 to 130°C;
• step e) is carried out using 1 to 3 mole of acid per mole of (IV), where the acid is selected from: HCl gas, aq. HCl, aq. H₂SO₄, CF₃CO₂H, CH₃CO₂H, oxalic acid, methanesulfonic acid or p-toluenesulfonic acid, at a temperature between 15 and 60°C, to obtain the compounds of formula (I) either as the salt of the acid used or as a free amine of formula (I), by addition of a tertiary amine, selected from triethylamine (TEA) or diisopropylethylamine (DIEA).

4. A process according to claims 1-3 for the preparation of (3β,5β,12α)-3-[[4(*S*)-4-amino-5-methoxy-1,5-dioxopentyl]amino]-12-hydroxycholan-24-oic acid methyl ester (I A): 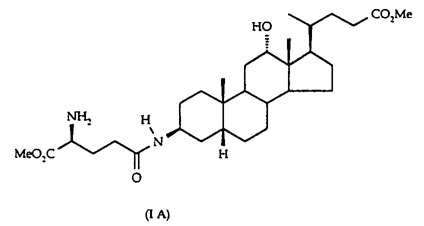 in which a suspension of one mole of compound (XI), (3β,5β,12α)-3-amino-12-hydroxycholan-24-oic acid methyl ester, 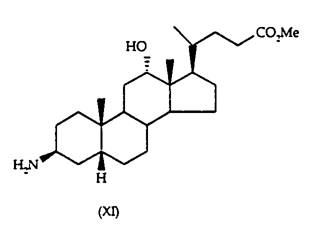 is subjected to transamidation in step d) by treatment with at least one mole of compound (V), in a solvent selected from: toluene, *N,N*-dimetilacetamide, p-cymene or diethylbenzene at a temperature from 80 to 110°C for 20 to 30 hour to afford the compound (VI A), (3β,5β,12α)-12-hydroxy-3-[[5-methoxy-1,5-dioxo-4(*S*)-4-[[(1,1-dimethylethoxy)carbonyl]amino]pentyl]-amino]cholan-24-oic acid methyl ester, by cooling the mixture to room temperature over 15 hours and filtering the precipitated compound; and
a solution of the compound of formula (VI A) is subjected to hydrolysis in step e) by slow addition of 1 to 3 moles of HCl or methanesulfonic acid solution, keeping temperature below 20°C, and the compound (I A) is finally isolated as a salt of the previously used acid, or as a free amine by neutralizing the solution by addition of a tertiary amine, selected from triethylamine (TEA) or diisopropylethylamine (DIEA).
